# EUROPEAN PATENT SPECIFICATION

(11) **EP 1 066 058 B1**
(45) Date of publication and mention of the grant of the patent: **10.12.2003**
(21) Application number: 99910879.8
(22) Date of filing: 19.03.1999
(51) Int. Cl.: A61K 39/395, A61P 37/00

(54) **IMMUNOTOXIN COCKTAILS FOR THE TREATMENT OF IMMUNE RELATED DISEASES**
IMMUNOTOXINMISCHUNGEN ZUR BEHANDLUNG VON IMMUNKRANKHEITEN.
COCKTAILS D'IMMUNOTOXINES POUR LE TRAITEMENT DE MALADIES D'ORIGINE IMMUNOLOGIQUE

(30) Priority: 23.03.1998 EP 98200917; 23.03.1998 US 79086 P
(43) Date of publication of application: 10.01.2001
(73) Proprietor: Immunotoko B.V., 6525 EC Nijmegen (NL)
(72) Inventor: VAN OOSTERHOUT, Ypke, Vincentius, Johannes, Maria, NL-6524 ML Nijmegen (NL); VAN EMST, Janny, Elisabeth, NL-6605 XA Wijchen (NL)
(74) Representative: Prins, Adrianus Willem
(86) International application number: NL9900156
(87) International publication number: WO99048534

(56) References cited:
- EP-A- 0 336 379
- WO-A-89/06967
- US-A- 5 045 451
- JANSEN, BURKHARD ET AL: "Successful treatment of human acute T-cell leukemia in SCID mice using the anti- CD7 -deglycosylated ricin A-chain immunotoxin DA7" CANCER RES. (1992), 52(5), 1314-21 CODEN: CNREA8;ISSN: 0008-5472,1992, XP002077287
- PREIJERS, F. W. M. B. ET AL: "Relationship between internalization and cytotoxicity of ricin A-chain immunotoxins" BR. J. HAEMATOL. (1988), 70(3), 289-94 CODEN: BJHEAL;ISSN: 0007-1048,1988, XP002077288 cited in the application
- PREIJERS, F. W. M. B. ET AL: "Human T lymphocyte differentiation antigens as target for immunotoxins or complement-mediated cytotoxicity" SCAND. J. IMMUNOL. (1988), 28(2), 185-94 CODEN: SJIMAX;ISSN: 0300-9475,1988, XP002077289
- PREIJERS, F. W. M. B. ET AL: "Different susceptibilities of normal T cells and T cell lines to immunotoxins" SCAND. J. IMMUNOL. (1988), 27(5), 533-40 CODEN: SJIMAX;ISSN: 0300-9475,1988, XP002077290 cited in the application
- VAN OOSTERHOUT Y V J M ET AL: "Suitability of a cocktail of CD34 and CD7 ricin A-immunotoxins for in vivo treatment of acute graft-versus-host-disease." THIRTY-NINTH ANNUAL MEETING OF THE AMERICAN SOCIETY OF HEMATOLOGY, SAN DIEGO, CALIFORNIA, USA, DECEMBER 5-9, 1997. BLOOD 90 (10 SUPPL. 1 PART 2). 1997. 376B. ISSN: 0006-4971, XP002077291
- P. HERVE ET AL.: "Depletion of T-Lymphocytes in Donor Marrow wit PAN-T Moabs and complement for prevention of acute graft-Versus-Host Disease: A Pilot Study on 29 Patients." J. NAT. CANCER INST., vol. 76, no. 6, June 1986, pages 1311-1316, XP002077292

## Description

The invention relates to the field of immune system related diseases, in particular to novel means and methods for treating these diseases. More in particular the invention provides novel means for eliminating or suppressing populations of unwanted CD3 and/or CD7 positive cells.

Typically the invention finds applications in the field of allogeneic bone marrow transplantation.

Allogeneic bone marrow transplantation (BMT) is a world-wide accepted method to treat a number of severe disorders like leukemia, myelo-dysplastic syndrome, bone marrow failure, immune deficiency, storage diseases and hemoglobinopathies (1-6). For a good engraftment, the bone marrow must contain a minimum number of T-cells (7-10). These cells may also confer benefit as they contribute to the so-called graft-versus-leukemia effect which involves the elimination of residual malignant cells (11-13). However, donor T cells may react with normal tissues of the host causing graft-versus-host-disease (GVHD) which results in serious damage to the skin, liver and gastro-intestinal tract (2,14-16). When this disease occurs within the first three months after BMT, it is classified as acute GVHD and when it develops at a later stage it is referred to as chronic GVHD. The severity of the clinical symptoms is expressed in four grades, grade I referring to minimal GVHD, and grade IV to the most severe form which is usually fatal and involves epidermiolysis, liver failure, and severe diarrhea (14).

The incidence and severity of GVHD can be diminished by depletion of T-cells from the graft. At the University Hospital Nijmegen, 98% of lymphocytes are depleted from the graft using counterflow centrifugation (17). However, despite depletion of the vast majority of T cells, GVHD can still occur. The annual incidence of grade 11-through IV GVHD at the University Hospital Nijmegen is - 6 out of a total of 40 allogeneic bone marrow transplant recipients. World-wide GVHD occurs in 30-70% of HLA-matched recipients and contributes to death in 20-40% of those affected (2,18). Even if a patient survives severe GVHD, this results in long-lasting disability and morbidity leading to repeated admission to hospital. The development of an effective alternative treatment for GVHD will therefore have a major impact on both survival and the quality of life of allogeneic BMT recipients.

Current treatment is usually as follows.

As immuno-prophylaxis cyclosporin is administered intravenously from one day before transplantation onwards (3 mg/kg/d for 15 days, and thereafter 2 mg/kg/d). As soon as the patient can take oral medication, cyclosporin is given orally (6 mg/kg/d). If the patient develops GVHD, 1st-line therapy in the form of corticosteroids is given (prednison: 1 mg/kg/d). In case the patient does not respond to this therapy within 48 hours, or if the GVHD is progressive within 24 hours, high-dose methylprednisolon (Solumedrol 4 x 250 mg/day) is given as second line therapy. When high-dose methylprednisolon has failed, patients are currently treated with Leukotac (a-IL2-receptor MoAb) (19). Although responses are seen in some cases during administration of Leukotac, GVHD normally recurs once treatment is discontinued. GVHD will eventually be fatal for about 90% of the patients not responding to the second line therapy within 3-5 days.

The present invention provides novel means and methods for among others the treatment of GvHD using molecules specifically recognizing receptors on cells to be eliminated. In a number of preferred embodiments at least one such a molecule will be coupled to a toxic moiety. Such molecules are often referred to immunotoxins.

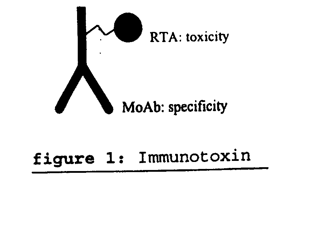

An immunotoxin (IT) is a conjugate of for instance a monoclonal antibody (MoAb), usually developed in a mouse, coupled to a potent toxin such as the A-chain of the potent plant toxin ricin (RTA) (figure 1 ) .

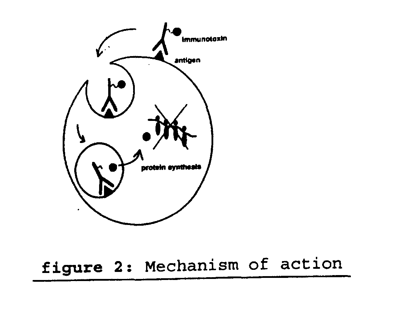

The mechanism of action of the IT is depicted in figure 2. The MoAb-moiety first binds specifically to an antigen expressed on those cells which are to be eliminated, where after the entire antigen-IT complex is internalized. Once inside the cell, the bond between the MoAb and the toxin is broken thereby releasing free toxin into the cytoplasm where it inhibits protein synthesis irreversibly by means of a catalytic reaction which culminates in the cell's death. Since the toxin-moiety is incapable of entering the cell autonomously and is inactive outside the cell, ITs are only hazardous to cells that express the specific target antigen and are capable of internalising the IT complex.

Since their introduction in 1979, ITs have been used to treat a variety of diseases including solid and diffuse tumors, immunological disorders and viral infections. At the Department of Hematology of the University Hospital Nijmegen, ITs have been investigated since 1984 within the framework of bone marrow transplantation to eliminate malignant T/B-cells from autologous bone marrow grafts and normal T cells from allogeneic bone marrow grafts (21-31).

Clinical results published so far are mainly from Phase I/II studies. The commonly observed Phase I dose-limiting toxicities of RTA-ITs are vascular leak syndrome (VLS) and myalgias. These side effects, due to the 'bystander' effects of the toxin, appeared to be transient and generally ceased shortly after immunotoxin administration was discontinued (32). Two different trials witnessed toxicity due to crossreactivity of the MoAb-part with undesired tissue (33,34). In both cases the IT, directed against ovarian carcinoma and breast carcinoma, respectively, crossreacted with neural tissue resulting in severe neurotoxicity. These observations stress the importance of extensive preclinical screening for crossreactivity with normal tissue. A common feature in most clinical trials was the development of human antibodies against both the MoAb and the toxin part of the IT (32). These humoral immune responses were not associated with enhanced toxicity nor allergic reactions, but decreased the serum half life of the IT. Nevertheless, clinical responses were seen even in the face of anti-IT antibody (35-37).

The efficacy of treatment with ITs, as observed in the Phase I trials, has shown to be strongly influenced by the accessibility of target cells. So far, treating solid tumors has met with little success whereas treatment of disorders like lymphomas and certain immunological diseases resulted in partial and complete responses in 12-75% of cases (38). These results are impressive considering the fact that treatment of cancer with >90% of the drugs currently available produced fewer than 5% of partial and complete responses in Phase I trials (38).

Thus the present invention provides the use of a pharmaceutical composition for eliminating or reducing the number of unwanted CD3 and/or CD7 positive cells, comprising a mixture of at least a first molecule specifically recognizing CD3 or CD7 and at least a second molecule specifically recognizing another ligand receptor associated with the surface of such an unwanted cell, whereby at least one of the specifically recognizing molecules is provided with a toxic moiety. A pharmaceutical composition is defined herein as any composition which can be administered to an individual, be it as one single dosis or as a regimen of doses by any viable route, preferably by intravenous administration, optionally containing usual vehicles for administration and/or components of regular treatment of the relevant immune related disease. Unwanted cells are any cells that comprise CD3 and/or CD7 (and of course many other) molecules associated with the cell surface, which cells are involved in a pathological condition in an individual. Typically these cells are T-cells or NK-cells or other cells playing a role in GvH or allograft rejection. Also aberrant cells (T cell leukemias or lymphomas for instance) comprising CD3 and/or CD7 (preferably both) can be eliminated or suppressed according to the invention.

A molecule specifically recognizing CD3 or CD7 or another ligand-receptor is a term well understood in the art. It means any molecule having a relatively high binding affinity and specificity for CD3, CD7 or the receptor. It may typically be a ligand for a receptor or an antibody for either CD3, CD7 or another receptor (receptor is defined as any molecule capable of a specific interaction), which antibody may be truncated or humanized or altered in any other way without losing its specificity (such alterations are herein defined as derivatives and/or fragments). Toxic moieties are basically any and all molecules that lead to toxicity for the target cell be it directly or indirectly, thus including but not limited to lectins, ricin, abrin, PE toxin, diphteria toxin, radio-isotopes, cytostatic drugs such as adriamycin, apoptosis inducing agents and prodrug converting substances together with prodrugs such as the thymidin kinase and gancyclovir combination. Preferred is Ricin A, preferably deglycosylated (In order to prevent the binding of ricin A to carbohydrate-receptors expressed by liver cells, deglycosylated ricin A (dgRTA) (20) is preferred). In prodrug embodiments typically one of the molecules specifically recognizing CD3 or CD7 or a receptor may be provided with the converting agent and the other with the prodrug. CD3 however does not need to be coupled to a toxic moiety to have an effect, since it blocks the interaction of the T cell receptor with APC's. This is one of the advantages of the present invention which are not present in the prior art.

As stated above it is preferred that both CD3 and CD7 are targeted by specific binding molecules therefor. This cocktail has proven itself in preliminary clinical studies, in an embodiment where the toxic moiety is attached to both specifically binding molecules, which are both antibodies. A surprising effect of this cocktail is that although in some cases GvH relapse occurs, this relapse is now treatable with low dose corticosteroids, which in first instance the GvH was not. Thus such a combination is another preferred embodiment of the present invention. The invention thus also provides a pharmaceutical composition as described above, whereby said first molecule specifically recognizes CD3 and said second molecule specifically recognizes CD7.

The toxic moiety may be coupled to the specifically binding molecule in any manner, such as by making a fusion protein by recombinant means, typically including a protease cutting site between binding molecule and toxic (protein) moiety, but for ease of manufacturing and freedom of choice in toxic moieties chemical coupling is preferred, optionally by an acid-labile linker. (Upon internalization a conjugate typically goes through a lysosome).

The invention also provides the use of a pharmaceutical composition according wherein at least two molecules specifically recognizing different receptors are provided with toxic moieties, which may be the same or different toxic moieties. There is a major advantage to using different toxic moieties when the side effects of the moieties are different, because higher doses can then be given. Typically the pharmaceutical compositions according to the invention may further comprise at least one further molecule specifically recognizing CD5, CD2, CD4, CD8 or an IL-2 receptor, which may also be coupled to a toxic moiety. This may provide higher efficacy, but may also be used to provide higher specificity for groups of cells, also in combination with for instance prodrug regimes.

It is preferred that when the molecule specifically recognizing CD3 is an antibody that the antibody is of the IgG gamma-2B class, because this antibody does not fix human complement or bind human Fc-receptors and, thereby, does not induce cytokine release by the targeted T cells. Thus the invention in yet another embodiment provides a pharmaceutical composition wherein said first molecule is a gamma2B IgG antibody or a derivative thereof, which recognizes CD3.

Doses used are given in the detailed description hereof. The limitas of doses of immunotoxins in regimens like the invented one are typically dependent on the immunotoxin, both because of the specificity and affinity of the specific binding molecules as well as because of the different tolerated doses for different drugs. Expressed in equivalents of Ricin A deglycosylated the limits will be generally within at least 25 micrograms per square meter body surface (a grown human of 80 kg, typically has 2 square meters of surface), preferably 100 micrograms of Ricin A per square meter of body surface. This is a lower limit of what may be given in one or more doses over one or more days of treatment. Generally the total dosis of Ricin A equivalents should not be higher than 25 mg per square meter body surface.

Typically the compositions according to the invention will be used for the preparation of a medicament for the treatment of Graft vs. Host disease, Graft rejections, T-cell leukemias, T-cell lymphomas or other CD3 and/or CD7 positive malignancies, autoimmune diseases or infectious immune diseases such as HIV-infection. As stated hereinbefore in GvHD and graft rejection a typical effect is seen in that after treatment with a composition as disclosed herein, the relapse is treatable with low doses of corticosteroids.

Such a regimen is thus also part of the invention.

Thus the invention also provides a kit of pharmaceutical compositions for treating Graft vs. Host disease and/or graft rejection comprising a composition according to anyone of claims 1-13 and a pharmaceutical composition comprising at least one corticosteroid. The invention leads to a drop in numbers in the population of unwanted cells to at least 20 % of the original amount, usually even to 5% or less. Typically this number stays low over a prolonged period of time in contrast to what prior art regimes have accomplished. The exemplified composition not only targets T cells, but also NK cells which is another advantage of the present invention.

### Detailed description of the invention

The rationale for ITs to treat GVHD is that these conjugates can be used for an efficient and specific eradicating of immunocompetent T cells responsible for the disease. In this perspective, ITs might be more effective and may cause less side effects than broadly immunosuppressive reagents as cyclosporine and corticosteroids. In 1990, Byers et al reported of a Phase I clinical trial in which they intravenously administered an anti-CD5 RTA-IT (Xomazyme-CD5) to treat corticosteroid-resistant GVHD (39). The initial results were very promising with skin, gastrointestinal tract, and liver disease responding in 73, 45, and 28% of cases, respectively (39). However, more recent clinical trials showed that Xomazyme CD5 was no more effective than alternative treatments (18). Consequently, the further development of Xomazyme-CD5 was abandoned.

Encouraged by the initial success of the IT-based treatment of GVHD, we set up to develop alternative ITs with superior anti-T cell activity. In order to achieve this, we conjugated RTA to a panel of MoAbs that react with antigens that are expressed (almost) exclusively on T cells namely, the T-cell differentiation-antigens CD3, CD5 and CD7, and have assayed each for its anti-T cell activity. From this preclinical study, it appears that a cocktail of SPV-T3a-RTA (CD3-IT) and WT1-RTA (CD7-IT) has the highest potential for treating patients with severe GVHD and that this mixture affords:
- **Synergistic cytotoxicity** in which the simultaneous incubation of half the effective individual dose of SPV-T3a-RTA and WT1-RTA is more effective in eliminating T cells than either IT alone (including the CD5-IT).
- **Broad mechanism of action** in which binding of SPV-T3a to the T-cell receptor/CD3 complex results in an additive immunosuppressive effect by blocking the recognition by the donor T cells of the foreign patient antigens. This effect is independent of action of ricin A. Moreover, the binding of this particular CD3-MoAb does not stimulate T cells to produce cytokines which would otherwise augment the severity of GVHD.
- **Broad spectrum reactivity** by which WT1-RTA is also reactive against NK-cells. These lymphocytes are thought to aggravate the severity of GVHD especially in the later phase of the disease.

It has been previously described that the use of combinations of ITs can strongly enhance the efficacy of target cell elimination. The most obvious advantage above single-IT-treatment is that fewer target cells will be multiple antigen-negative than single antigen-negative. In addition, those cells which do express substantial levels of all target-antigens might be loaded with IT to a higher extent. When the respective IT follow a different intracellular routing, the change of escaping therapy might be further reduced. With respect to the use of anti-T-cell IT, reports addressing the combination or cocktail approach are thus far focused on in vitro applications, including the purging of bone marrow grafts. For one aspect of the present invention, we state that a mixture of murine mAb SPV-T3a (CD3, IgG2b) and WT1 (CD7, IgG1), both conjugated to dgA, forms a superior combination for the elimination or suppression of unwanted (e.g. over-reactive, misdirected, or malignant) T cells and/or NK cells. This particular combination affords important benefits which do surpass the 'common' synergism as observed with the more or less 'random' combinations of anti-T cell IT.

Some aspects delineating the superior characteristics of this particular combination are described below
(A) Antigen binding of a CD3 or a T-cell receptor (TCR) mAb results in an at least partial blocking and modulation (internalization or shedding) of the CD3/TCR-complex and thereby preventing alloactivation of the T lymphocytes (page 1, appendix III).
(B) Binding of a CD3 or a TCR mAb results in an at least partial Fas-mediated apoptosis of a significant fraction of activated T lymphocytes, according to a mechanism described as activation induced cell death (AICD) (see also page 2, appendix III).
   These two effector mechanisms, which are independent of a conjugated toxin, are of vital importance when intervening in an acute life-threatening situation as refractory GVHD. The temporal (blocking and modulation of CD3/TCR) as well as limited (AICD) nature of these effects only stresses the benefit of making a 'real killer' of the mAb by conjugating it to a toxin. One reason why we choose for SPV-T3a as CD3 mAb is that SPV-T3a is of IgG2b-isotype and has proven not to induce cytokine release by T cells isolated out of the majority of the human population examined. As a consequence, the risk of the so called 'cytokine release syndrome', which severely complicates the immunological disorder to be treated, is strongly reduced.
(C) The presence of the CD7-IT in the IT-cocktail is essential, apart from the above mentioned 'common' synergism (see also page 3, appendix III), in that it broadens the spectrum reactivity of the IT-cocktail. The CD7 antigen is also expressed on NK cells, which accordingly form a target for this particular IT-cocktail as well (see also page 4, appendix III). The significance of this is stressed by our changed insight regarding the processes underlying GVHD. In our view, NK cells play a distinctive role in the pathophysiology of GVHD, particularly in the efferent phase of the disease.

### 2.4 Clinical history of the IT-cocktail components:

***SPV-T3a:*** SPV-T3a is a mouse IgG2b MoAb directed against the human T-cell differentiation antigen CD3 (40).

Anti-CD3 antibody therapy is often associated with the cytokine release syndrome caused by the binding to the T-cell receptor/CD3 complex (41-44). One of the important benefits of SPV-T3a is that this particular MoAb does not induce cytokine release because of its IgG2b-isotype (45,46).

At the Department of Hematology of the University Hospital Nijmegen part of the bone marrow obtained from HLA-matched unrelated donors is currently treated *ex vivo* with a cocktail of SPV-T3a-RTA and WT1-RTA in order to eliminate immunocompetent T cells. The patients transplanted with this marrow showed normal hematopoetic reconstitution without any signs of toxicity (n = 3, data not shown).

***WT1:*** MoAb WT1 is a mouse MoAb of IgG2a isotype directed against the human T cell differentiation antigen CD7 (47, 48) .

At the Department of Nephrology of the University Hospital Nijmegen three patients which underwent a kidney-transplantation, have been treated with WT1 in order to treat an acute rejection. The administration of unconjugated WT1 appeared to be safe and did not result in allergic reactions nor in severe toxicities (Dr W Tax, personal communication). No clinical efficacy could be noted.

WT1 has been conjugated to dgRTA and administered to rhesus monkeys to test its suitability for use in the therapy of leukemic meningitis (49). The major conclusion of this study was that WT1-dgRTA may be safely administered intrathecally to rhesus monkeys and could be a good candidate for the treatment of T-lymphoblastic CNS leukemia.

At the Department of Hematology of the University Hospital Nijmegen, WT1-RTA is used since 1986 for the *ex vivo* purging of autologous BM of patients suffering from high-risk T-cell leukemia/lymphoma in order to eliminate residual malignant cells. After purging, neither neutrophil engraftment nor immunological reconstitution was delayed (n = 20) (25).

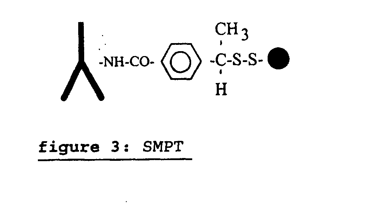

***SMPT cross-linker:*** The MoAbs are conjugated to dgRTA using the chemical cross-linker SMPT (figure 3). The cross-linker contains a disulfide bond which is important for the intracellular dissociation of the MoAb and dgRTA (necessary for toxicity, see figure 2).

SMPT is a so called 'second-generation cross-linker', characterized by having a hindered disulfide bond (due to the presence of the phenyl ring). This renders the SMPT-linker less susceptible to *extracellular* reduction by thiols present in the tissues and blood, and, therefore, results in a prolonged serum half-live of the IT. Thorpe et al demonstrated in an *in vivo* mice tumor model that using SPMT instead of the first-generation cross-linker SPDP, strongly improves the anti-tumor effect of their dgRTA-based ITs (20). Amlot et al performed a Phase I trail in which they studied the treatment of malignant lymphoma by intravenous administration of a SMPT-conjugated IT (RFB4[IgG]-dgRTA) (50) Due to the long serum half-live of 7.8 hours, therapeutic serum concentrations could be maintained between the infusions given at 48-hours intervals.

***dgRTA:*** The earliest RTA-based ITs consisted of a MoAb conjugated to native RTA. The oligosaccharides present on the native RTA, resulted in rapid hepatic clearance and hepatotoxicity *in vivo* (20, 51). This problem has been addressed in the second-generation ITS which make use of either deglycosylated RTA (dgRTA) or non-glycosylated recombinant ricin A (rRTA) (52, 53).

Vitetta and colleagues have reported the administration of dgRTA-based ITs to patients with refractory B-cell non-Hodgkin's lymphoma. They tested two different constructs. In the first, the Fab' fraction of MoAb RFB4 (anti-CD22) was conjugated to dgRTA (50). In the later construct they used RFB4 whole molecule (54).

The ITs were administered by 4-hour intravenous infusions given at 48-hours intervals. The Phase I dose limiting toxicities included pulmonary edema, expressive aphasia, and rhabdomyolysis with acute renal failure. Other side effects included hypoalbuminemia, weight gain, fever, tachycardia, decrease in electrocardiogram voltage, myalgias, anorexia, and nausea. The maximum tolerated dose (MTD) was 75 mg/m² for the Fab'-dgRTA and 32 mg/m² for whole IgG-dgRTA, which appeared to be inversely related to the serum half life (86 minutes and 7.8 hours, respectively). The two forms of the dgRTA-IT demonstrated no significant difference in clinical responses (partial and complete responses in 45% of the patients receiving > 50% of the MTD), in immunogenicity or in the toxic side effects. Because of its lower costs, the IgG-dgRTA IT was selected for further development.

The major findings to be learned from these studies are: *a)* The MTD of dgRTA-ITs is dependent primarily on the size of an individual dose rather than the cumulative dose. When administering RFB4(IgG)-dgRTA at 48-hours intervals at doses of 8 mg/m² or less, only grade I or II toxicities were observed. Total doses of 32 mg/m² RFB4(IgG)-dgRTA were consistently safe. As a consequence of the relative long T1/2, therapeutic serum concentrations (~ 1.8 µg/ml) could be maintained during and between infusions *b)* Side effects of the dgRTA-ITs administration were relatively modest and consisted predominantly of VLS and myalgia. No hepatotoxicity and minimal BM toxicity was observed. *c)* Patients with underlying pulmonary disease should not be treated because of the danger of VLS contributing to further pulmonary insufficiency.

Patients received four doses of IT-cocktail administered intravenously in 4-hours infusions at 48-hours intervals. If no clinical response is observed and if no severe toxicities (grade III or IV) occur, the study is continued with the next higher protein dose level.

### 4.0 Patient population

Patients have received 2^{nd}-line high dose corticosteroid therapy (methylprednisolon 1000 mg/d) for at least three days without any decrease of the severity of aGVHD.

Patients are EXCLUDED from participation in the study if:
1. The patient has a significant history or current evidence of intrapulmonary disease.
2. The patient has history of allergy to mouse immunoglobulins or ricin.
3. The patient has circulating high levels of human anti-mouse antibodies (HAMA).

### 5.0 Treatment

### 5.1 Pharmacological information:

The IT-cocktail has been prepared by the Department of Hematology under supervision of the Department of Clinical Pharmacy of the University Hospital Nijmegen. The IT-cocktail is stored at -80°C at 1 mg/ml in 0.15 M NaCl, in lots of 5 and 20 mg. Before infusion, the IT-cocktail will be filtered through a 0.22 µm filter and diluted to a final volume of 100 ml in normal saline solution. The ID₅₀ against the T cell line Jurkat is taken as the standard for biological activity when evaluating the quality of different lots of IT-cocktail.

### 5.2 lmmunotoxin administration:

Immunotoxins are administered via a central venous catheter. Prior to therapy, patients are given an i.v. test done with 200 µg IT-cocktail. Therapy is only started in the absence of anaphylactoid reactions. The IT-cocktail is administered in four doses at 48-hours intervals. The rationale of this is to give all the IT-cocktail before any host antibody response is expected to arise (usually not before 10 to 14 days after administration of xenogenic Ig).

The patient is initially treated with two subsequent doses of 2 mg/m². At this dose level no side effects are observed. In the absence of grade III or IV toxicities, the dose will be enhanced to 4 mg/m² if necessary..

### 5.3 Guidelines for dose modification:

Toxicities related to the immunotoxin administration are graded as grade I (mild), II (moderate), III (severe) or IV (life threatening) based on World Health Organization (WHO) guidelines (Appendix V). Special attention must be paid to the vascular leak syndrom (VLS). The physical signs of VLS being weight gain, peripheral edema, decrease in blood pressure, hypoalbuminemia, and small pleural effusions.

***Consecutive doses given to the same patient*:** Infusion of the second, third and fourth dose at any dose level is dependent upon the toxicity achieved after the previous infusion:
Grade I toxicity: no change in the scheduled dosage.
Grade II toxicity: 24 hours-delay of the dosage, with the next dose given if toxicity improves.
Grade III toxicity: the next dosage of immunotoxin will be withheld and only given if toxic parameters have improved (halving of the dosage can be considered).
Grade IV toxicity: no further dosage.

***Dose escalation:*** Progression from one dose level to the next should only occur after:
- The patient(s) of the group treated with the previous dose level have received four doses of IT-cocktail and have been observered for at least 48 hours after the last dosis, and,
- Dose limiting toxicity has not been reached.

***Dose limiting toxicity:*** Dose limiting toxicity is defined as the occurrence of adverse reactions of grade III or IV in an individual patient. If two patients experience a Grade III toxicity or if Grade IV toxicity occurs in a single patient, three additional patients will be entered at this dose level. If non of these additional patients demonstrate toxicity of grade III or IV, administration will again be continued to the next higher dose level. If Grade IV toxicity occurs in two patients at a given dose, the next patients will be treated with the previous dose level which will be considered the Maximum Tolerable Dose (MTD).

### 5.4 Concomitant medication and treatment:

Immunosuppressive agents used for prophylaxis and initial treatment may maintain unchanged throughout immunotoxin therapy.

### 6.1 Pretreatment studies:

Before entry into the study the patient undergoes a general examination consisting of medical history, physical examination with special emphasis on acute GVHD, measurement of oxygen saturation, electrocardiogram (ECG), and chest x-ray. The laboratory measurements will include Na+, K+, Cl-, HCO₃-, urea, creatine, billirubin, glucose, AP, ASAT (GOT), ALAT (GPT), gGT, LDH, total protein plus electrophoresis, leukocytes plus differentiation, red cells, hemoglobin, hematocrit, thrombocytes.

The patient is assayed for human anti-mouse and anti-ricin responses (HAMA/HARA), and serum levels of IL-2, TNF-a and IFN-g. Furthermore a quantitative alloreactive T-helper/T-cytocoxic precursor assay will be performed (see Appendix I).

### 6.1 Follow-up studies:

Daily complete physical examination is performed during IT-cocktail administration until 2 days after the last dose and then weekly thereafter. Blood is analyzed daily for Na+, K+, Cl-, HCO3-, urea, creatine, glucose, albumin, leukocytes plus differentiation, red cells, hemoglobin, hematocrit, and thrombocytes. Every two days for billirubin, AP, ASAT (GOT), ALAT (GPT), gGT, LDH, total protein plus electrophoresis (see Appendix I).

Vital signs (blood pressure, pulse, respiration frequention, and temperature) are checked every 15 minutes during the 1st hour post infusion, every 30 min during the 2nd up to 4th hour post infusion and from then on every hour up to eight hours post infusion. Besides, vital signs are assessed daily during IT-cocktail administration until 2 days after the last dose and then weekly thereafter.

***Pharmocokinetics and clearance of ITs:*** Blood samples are collected at 0, 1, 3, 4, 8, 12, 24, and 48 hours after each infusion. The serum concentrations of SPV-T3a-dgRTA and WT1-dgRTAare quantitatively determined in a sensitive and mAb-isotype specific immuno-radiometric assay (IRMA). From these results the individual serum halflifes of the two is calculated using the non linear least squares regression analysis.

***Measurement of humoral responses:*** In order to examine HAMA and HARA responses, serum samples are obtained one day pre-injection and subsequently weekly after the first infusion until the patient comes off study. The concentration of HAMA and HARA is determined in a sensitive radiometric assay.

***Immunological monitoring:*** Blood is sampled every other day during IT-cocktail administration until 2 days after the last dose and then weekly thereafter for immunological monitoring. PBLs are isolated and evaluated for composition by flow cytometry using antibodies reacting specifically with T cells subsets, B-cells, monocyte/macrophages, and NK cells. Serum are collected to determine levels of IL-2, TNF-a and IFN-g by commercial enzyme-linked immunosorbent assay (ELISA) kits.

The proliverative and cytotoxic activity of alloreactive PBLs is tested 2 days following the last dose using standard T-helper and T-cytotoxic-precursor assays, respectively.

### Staging and Grading of GVHD and clinical responses:

GVHD is scored daily during IT-cocktail administration until 2 days after the last dose and then weekly thereafter until the patient comes off study. Each organ system is staged grade 1 through 4 aGVHD according to the criteria of Glucksberg et al (Appendix II): skin by amount of surface involved with rash, gastrointestinal tract by the volume of diarrhea, and liver by serum bilirubin levels. Patients are also given an overall grade of aGVHD based on severity of organ involvement (Appendix II).

Responses to therapy are defined as follows:
- complete response (CR): the disappearance of symptoms in all organ systems;
- partial response (PR): improvement of ³1 organ, with no worsening in other organs.
- mixed response (MR): improvement of ³1 organ, with worsening in ³1 other organ.
- stable disease: no significant change in any organ system.
- progressive disease (PD): progression in ³1 organ-system without improvement in any organs.

The duration of response is defined as the period from the date the response was first recorded to the date on which subsequent progressive disease is first noted.

### Results.

Animal toxicity studies and preclinical studies.

Animal toxicity studies and preclinical studies are summarized in appendix III.

### Clinical study.

The results of the clinical study are reported in Appendix IV.

### Materials and methods.

### Making monoclonal antibodies.

Monoclonal antibodies disclosed herein are prepared using hybridoma technology well known in the art. Selection steps to select immunoglobulins having the properties disclosed hereinabove are also well known in the art and may include affinitychromatography and the like.

### Preparation of F(ab')2 fragments.

F(ab')₂ fragments of antibodies were prepared using a "F(ab')₂ preparation kit" (Pierce, Rockford, IL), according to the manufacturers' protocol. Briefly, antibodies were incubated with immobilized pepsin at pH 4.2 (20 mM sodium acetate buffer) for 4, and 16 h, respectively. Undigested IgG molecules, and Fc-fragments were removed by affinity chromatography with protein A-sepharose. Remaining fragments smaller than 30 kD were removed by means of a centriprep-30 concentrator (Amicon, Beverly, MA). The purity of F(ab')₂ fragments was determined by SDS-PAGE, which revealed less then 1% contamination with either intact IgG or Fc-fragments (data not shown).

### IT preparation.

Antibodies were conjugated on a 1 to 1 ratio (m/m) to deglycosylated ricin A (dgA, Inland Laboratories, Austin, Texas) using the SMPT-crosslinker (Pierce, Rockford, IL), according to the method as described by Ghetie et al. (Ghetie V et al. The GLP large scale preparation of immunotoxins containing deglycosylated ricin A chain and a hindered disulfide bond. J Immunol Methods 1991, 142: 223-30).

Antibodies were conjugated to ricin A (kindly provided by Dr. F.K. Jansen; Centre de Recherches Clin Midy, Montpellier, France) using N-succinimydyl 3-(2-pyridyldithio)propionate (SPDP; Pharmacia) or SMPT, as described.(The conjugation ratios of ricin A to mAb were estimated by measurement of absorbance at 280 nm and RIA, and were determined to be in the order of 0.8 to 1.2. Preservation of antibody-binding activity following conjugation was assessed by FCM.

### REFERENCES

1. Storb R, Thomas ED: Human marrow transplantation. Transplantation 1979, 28: 1.
2. O'Reilly RJ. Allogeneic bone marrow transplantation: current status and future directions. Blood 1983, 62: 941.
3. Goldman JM, Apperley JF, Jones L, et al. Bone marrow transplantation for patients with chronic myeloid leukemia. N Engl J Med 1986, 314: 202.
4. Champlin R. Bone marrow transplantation for acute leukemia: a preliminary report from the International Bone Marrow Transplant Registry. Transplant Proc 1987, 19: 2626.
5. Ringden O, Sundberg B, Lonnqvist B, et al. Allogeneic bone marrow transplantation for leukemia: factors of importance for long-term survival and relapse. Bone Marrow Transplant 1988, 3: 281.
6. Snyder DS, Findley DO, Forman SJ, et al. Fractionated total body irradiation and high dose cyclophoshamide: a preparative regimen for bone marrow transplantation for patients with hematologic malignancies in first complete remission. Blut 1988, 57: 7.
7. Martin PJ, Hansen JA, Buckner CD, et al. Effects of in vitro depletion of T cells in HLA-identical allogeneic marrow grafts. Blood 1985, 66: 664.
8. Patterson J, Prentice HG, Brenner MK, et al. Graft rejection following HLA matched T-lymphocyte depleted bone marrow transplantation. Br J Haematol 1986; 63: 221.
9. Kernan NA, Flomenberg N, Dupont B, et al. Graft rejection in recipients of T cell-depleted HLA-nonidentical marrow transplants for leukemia. Transplantation 1987, 43: 842.
10. Vallera DA, Blazar BR. T cell depletion for graft-versus-host-disease prophylaxis. A perspective on engraftment in mice and humans. Transplantation 1989, 47: 751.
11. Horowitz M, Gale RP, Sondel et al. Graft-versus-leukemia reactions after bone marrow transplantation. Blood 1990, 75: 555.
12. Sullivan KM, Weiden PL, Storb R, et al. Influence of acute and chronic graft-versus-host disease on relapse and survival after bone marrow transplantation from HLA-identical siblings as treatment of acute and chronic leukemia. Blood 1989, 73: 1720.
13. Truitt RL, Lefevre AV, Shih CC, Graft-vs-leukemia reactions: Experimental models and clinical trials. In: Gale RP, Champlin R (eds). Progress in bone marrow transplantation, New York, Liss. 1987: 219.
14. Glucksberg H, Storb R, Fefer A, et al. Clinical manifestations of graft-versus-host disease in human recipients of marrow from HLA-matched sibling donors Transplantation 1974, 18: 295.
15. Grebe SC, Streilein JW. Graft-versus-host disease. Adv Immunol 1976, 22: 119.
16. Ferrara JLM, Deeg HJ. Graft-versus-host disease. N Engl J Med 1991, 304: 667.
17. De Witte T, Hoogenhout J, De Pauw B, et al. Depletion of donor lymphocytes by counterflow centrifugation successfully prevents acute graft-versus-host disease in matched allogeneic marrow transplantation. Blood 1986, 67: 1302.
18. Hings IM, Severson R, Filipovich AH, et al. Treatment of moderate and severe acute GVHD after allogeneic bone marrow transplantation. Transplantation 1994, 58: 437.
19. Hervé P, Wijdenes J, Bergerat JP, et al. Treatment of corticosteroid-resistant acute graft-versus-host disease by in vivo administration of anti-interleukin-2 receptor monoclonal antibody (B-B10). Blood 1990, 75: 1426.
20. Thorpe PE, Wallace PM, Knowles PP, et al. Improved anti-tumor effects of immunotoxins prepared with deglycosylated ricin A chain and hindered disulfide linkages. Cancer Res 1988, 48: 6396.
21. Preijers FWMB, Tax WJM, Wessels JMC, et al. Different susceptibilities of normal T cells and T cell lines to immunotoxins. Scand J Immunol 1988a, 27: 533.
22. Preijers FWMB, Tax WJM, De Witte TJM, et al. Relationship between internalization and cytotoxicity of ricin A-chain immunotoxins. Br J Haematol 1988b, 70: 289.
23. Preijers FWMB, De Witte T, Rijke-Schilder GPM, et al. Human T lymphocytes differentiation antigens as target for immunotoxin or complement-mediated cytotoxicity. Scand J Immunol 1988c, 28: 185.
24. Preijers FWMB, De Witte T, Wessels JMC, et al. Cytotoxic potential of anti-CD7 immunotoxin (WT1-ricin A) to purge ex vivo malignant T cells in bone marrow. Br J Haematol 1989a, 71: 195.
25. Preijers FWMB, De Witte T, Wessels JMC, et al. Autologous transplantation of bone marrow purged *in vitro* with an anti-CD7-(WT1)-ricin A immunotoxin in T cell lymphoblastic leukemia and lymphoma. Blood 1989b, 74: 152.
26. Preijers, FWMB. Rationale for the clinical use of immunotoxins: monoclonal antibodies conjugated to ribosome-inactivating proteins. Leukemia Lymphoma 1993, 9: 293
27. Van Horssen PJ, Van Oosterhout YVJM, De Witte T, et al. Cytotoxic potency of CD22-ricin A depends on intracellular routing rather than on the number of internalized molecules. Scand. J Immunol 1995, 41: 563.
28. Van Oosterhout YVJM, Preijers FWMB, Wessels JMC, et al. Cytotoxicity of CD3-ricin A chain immunotoxins in relation to cellular uptake and degradation kinetics. Cancer Res 1992, 52: 5921.
29. Van Oosterhout YVJM, Van De Herik-Oudijk IE, Wessels HMC, et al. Effect of isotype on internalization and cytotoxicity of CD19 ricin A immunotoxins. Cancer Res 1994a, 54: 3527.
30. Van Oosterhout YVJM, Preijers FWMB, Meijerink JPP, et al. A Quantitative flow cytometric method for the determination of immunotoxin-induced cell kill in marrow grafts. Advances in bone marrow purging and processing: Fourth international symposium 1994b, pages 89-95, Wiley-Liss, Inc.
31 Van Oosterhout YVJM, Van Emst L, De Witte T, et al. Suitability of a cocktail of CD3- and CD7-ricin A immunotoxins for *in vivo* treatment of GVHD. Abstract for the Fourth International Symposium on Immunotoxins: June 8-10, 1995, Myrtle Beach, South Carolina.
32. Ghetie V, Vitetta E. Immunotoxins in the therapy of cancer from bench to clinic. Pharmac Ther 1994, 63: 209.
33. Weiner LM, O'Dwyer J, Kitson J, et al. Phase I evaluation of an anti-breast carcinoma monoclonal antibody 260F9-recombinant ricin A conjugate. Cancer Res 1989, 49: 4062.
34. Pai LH, Bookman MA, Ozols RF, et al. Clinical evaluation of intraperitoneal Pseudomonas exotoxin immunoconjugate of OVB3-PE in patients with ovarian cancer. J Clin Oncol 1991, 9: 2095.
35. Oratz R, Speyer JL, Werntz JC, et al. Antimelanoma monoclonal antibody-ricin A chain immunoconjugate (XMMME-001-RTA) plus cyclophosphamide in the treatment of metastatic malignant melanoma: results of a Phase II trial. J biol Resp Mod 1990, 9: 345.
36. LeMaistre CF, Rosen S, Frankel A, et al. Phase I trial of H65-RTA immunoconjugate in patients with cutaneous T-cell lymphoma. Blood 1991, 78: 1173.
37. LeMaistre CF, Deisseroth A, Fogel B, et al: Phase I trial of an interleukin-2 (IL-2) fusion toxin (DAB₄₈₆IL-2) in hematologic malignancies expressing the IL-2 receptor. Blood 1992, 79: 2547.
38. Vitetta E, Thorpe PE, Uhr JW. Immunotoxins: magic bullets or misguided missiles? Immunol Today 1993, 14: 252.
39. Beyers VS, Henslee PJ, Kernan NA, et al. Use of an anti-pan T-lymphocyte ricin A chain immunotoxin in steroid-resistant acute graft-verus-host disease. Blood 1990, 75: 1426.
40. Spits H, Keizer G, Borst J, et al. Characterization of monoclonal antibodies against cell surface molecules associated with cytotoxic activity of natural and activated killer cells and cloned CTL lines. Hybridoma 1983, 2: 423.
41. Thistlethwaite JR, Stuart JK, Mayes JT, et al. Complications and monitoring of OKT3 therapy. Am J Kidney Dis 1988, 11: 112.
42. Ellenhorn JDI, Woodle ES, Ghobrial I, et al. Activation of human T cells in vivo following treatment of transplant reicpients with OKT3. Transplantation 1990, 50: 608.
43. Abramowicz D, Schandene L, Goldman M, et al. Release of tumor necrosis factor, interleukin-2, and gamma-interferon in serum after injection of OKT3 monoclonal antibody in kidney transplant recipients. Transplantation 1989, 47: 606.
44. Chatenoud L, Ferran C, Legendre C, et al. In vivo cell activation following OKT3 administration. Transplantation 1990, 49: 697.
45. Woodle ES, Thistlethwaite JR, Jolliffe LK, et al. Anti-CD3 monoclonal antibody therapy. Transplantation 1991, 52: 361.
46. Frenken LAM, Koene RAP, Tax WJM. The role of antibody isotype in IFN-g and IL-2 production during anti-CD3-induced T cell proliferation. Transplantation 1991, 51: 881.
47. Tax WJM, Willems HW, Kibbelaar MDA, et al. Monoclonal anibodies against human thymocytes and T lymphocytes. Protides of the biological fluids, 29th Colloquium 1981, edited by Peeters H, Pergamon Press, Oxford and New York, 1982.
48. Tax WJM, Tidman N, Janossy G, Trejdosiewicz L, Willems R, Leeuwenberg J, De Witte TJM, Capel PJA, Koene RAP: Monoclonal antibody (WT1) directed against a T cell surface glycoprotein: characteristics and immunosupressive activity. Clin Exp Immunol 55: 427, 1984.
49. Hertler AA, Schlossman DM, Borowitz MJ, et al. An immunotoxin for the treatment of T-acute lymphoblastic leukemic meningitis: studies in rhesus monkeys. Cancer Immunol Immunother 1989, 28: 59.
50. Amlot PL, Stone MJ, Cunningham D, et al. A phase I study of an anti-CD22-deglycosylated ricin A chain immunotoxin in the treatment of B-cell lymphomas resistant to conventional therapy. Blood 1993, 82: 2624-2633.
51. Blakey DC, Watson GJ, Knowles PP, et al. Effect of chemical deglysocylation of ricin A chain on the in vivo fate and cytotoxic activity of an immonotoxin composed of ricin A chain and anti-Thy 1.1 antibody. Cancer Res 1987, 47: 947.
52. Ghetie V, Ghetie M, Uhr JW, et al. Large scale preparation of immunotoxins constructed with the Fab' fragment of IgG1 murine monoclonal antibodies and chemically deglycosylated ricin A chain. J immun Meth 1988, 112: 267.
53. Bjorn MJ, Ring D, Frankel A. Evaluation of monoclonal antibodies for the development of breast cancer immunotoxins. Cancer Res 1985, 45: 1214.
54. Vitetta ES, Stone M, Amlot P, et al. Phase I immunotoxin trial in patients with B-cell lymphoma. Cancer Res 1991, 51: 4052.

**Table I:**

| Dose levels: | | | | | |
|---|---|---|---|---|---|
| | Dosage of IT-cocktail (mg/m²) | | | | |
| # Patients | d1 | d3 | d5 | d7 | Total |
| 1 | 2 | 2 | 4 | 4 | 12 |
| 2 | 4 | 4 | 4 | 4 | 16 |
| 2 | 8 | 8 | 8 | 8 | 32 |
| 2 | 10 | 10 | 10 | 10 | 40 |

### Appendix 1: Flow-chart of IT-cocktail study (summary of study requirements):

| | **PROTOCOL FLOW SHEET** **(days following initiation of the study)** | | | | | | | | | | | | | | |
|---|---|---|---|---|---|---|---|---|---|---|---|---|---|---|---|
| | | 1st inj . ↓ | | 2nd inj . ↓ | | 3rd inj . ↓ | | 4th inj. ↓ | | | | | | | |
| | Pre | d1 | d2 | d3 | d4 | d5 | d6 | d7 | d8 | d9 | d10 | d22 | d29 | d36 | d43 |
| Informed consent | o | | | | | | | | | | | | | | |
| History and Phys. exam. | o | o | o | o | o | o | o | o | o | o | o | o | o | o | o |
| Chest x-ray | o | | | | | | | | | | | | | | |
| ECG | o | | | | | | | | | | | | | | |
| Vital signs | | o¹ | o | o¹ | o | o¹ | o | o¹ | o | o | o | o | o | o | o |
| Biochemistry² | o | o | o | o | o | o | o | o | o | o | o | o | o | o | o |
| Hematology³ | o | o | o | o | o | o | o | o | o | o | o | o | o | o | o⁴ |
| Pharmokinetics | | o⁵ | | o⁵ | | o⁵ | | o⁵ | | | | | | | |
| HAMA/HARA | o | | | | | | | | o | | o | o | o | o | o |
| Flowcytometry ⁶ | o | | o | | o | | o | | o | o | o | o | o | o | o |
| Cytokine levels ⁷ | o | | o | | o | | o | | o | o | o | o | o | o | o |
| Allo-precursors | o | | | | | | | | o | | | | | | |
| GVHD staging | o | o | o | o | o | o | o | o | o | o | o | o | o | o | o ⁸ |

| | | | | | | | | | | | | | | | |
|---|---|---|---|---|---|---|---|---|---|---|---|---|---|---|---|
| 1: Vital signs are checked every 15 min during the 1st hour post-injection, every 30 min during the 2nd up to the 4th hour, and from then on every hour up to 8 hours post-injection. | | | | | | | | | | | | | | | |
| 2: The pre-study biochemistry panel includes Na+, K+, Cl-, HCO₃-, urea, creatine, billirubin, glucose, AP, ASAT (GOT), ALAT (GPT), γGT, LDH, and total protein plus electrophoresis. During the follow-up study, blood will be analyzed daily for Na+, K+, Cl-, HCO₃-, urea, creatine, glucose,and albumin. Besides, every two days is added billirubin, AP, ASAT (GOT), ALAT (GPT), γGT, LDH, and total protein plus electrophoresis. | | | | | | | | | | | | | | | |
| 3: The hematology panel includes leukocytes plus differentiation, red cells, hemoglobin, hematocrit, thrombocytes. | | | | | | | | | | | | | | | |
| 4: To be continued weekly until WBC numbers have returned to normal. | | | | | | | | | | | | | | | |
| 5: Venous blood samples are obtained pre-injection and 1, 3, 4, 8, 12, 24, and 48 h after each injection. Besides, a sample is taken 72 h following the last injection. | | | | | | | | | | | | | | | |
| 6: The flowcytometry panel includes the markers CD2, CD3, CD4, CD5, CD7, CD8, CD14, CD19 and CD56. | | | | | | | | | | | | | | | |
| 7: Serum is assayed for levels of IL-2, TNF-α and IFN-γ. | | | | | | | | | | | | | | | |
| 8: To be continued monthly when responses are observed. | | | | | | | | | | | | | | | |

### Appendix II: staging acute graft-versus-host disease

(Glucksberg et al, Transplantation 1974, 18: 295-304)

### Skin

grade 1: maculo-papular eruption involving less than 25% of the body surface
grade 2: maculo-papular eruption involving less than 25%to 50% of the body surface
grade 3: generalized erythroma

### Liver

grade 1: bilirubin 34-50 µmol/l
grade 2: bilirubin 51-100 µmol/l
grade 3: bilirubin 101-254 µmol/l
grade 4: ³255 µmol/l

### Gut

grade 1: 500 to 1000 ml of stool/day
grade 2: 1001 to 1500 ml of stool/day
grade 3: 1501 to 2000 ml of stool/day
grade 4: >2000 ml of stool/day

### Overall severity of aGVHD

grade 1: skin: 1/2; gut: 0; liver 1
grade 2:skin: 1/2/3/; gut 1/2 and/or liver 1/2 sometimes associated with fever
grade 3 skin: 2/3/4; gut 2/3/4 and or liver 2/4 often associated with fever.
grade 4: simular to grade 3 but extreme constitutional symptoms

### INHIBITION OF ALLOACTIVATION BY UNCONJUGATED SPV-T3a

### Method:

Alloactivation was analyzed in a mixed lymphocyte culture (MLC). MLC were performed with 'responder' peripheral blood lymphocytes (PBL) mixed in a one to one ratio with irradiated 'stimulator' PBL. Cultures were performed in triplicate (5x10⁴ cells/well) in U-bottemed microtiter plates in 150 µl culture medium at 37°C and 5% CO₂. Prior to, or at different days following initiation of the MLC, SPV-T3a (10⁻⁸ M) or an irrelevant isotype-matched control antibody were added to the culture medium. Following 72 h of culture, plates were labeled with [³H]thymidine (0.4 µCi/well) for 4 h. Subsequently, the proliferation of responder cellls was determined by collecting the DNA using a cell harvester and counting the incorporated radioactivity. Proliferation was expressed as percentage of the untreated control.

### Results:

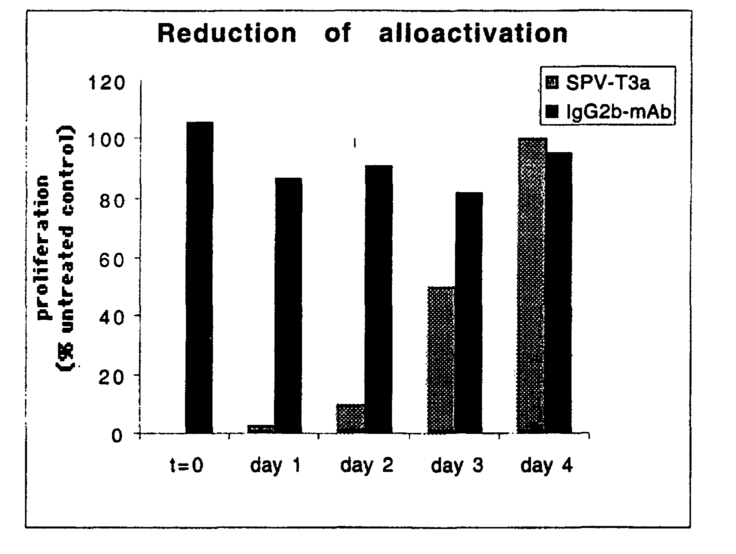

Alloactivation was completely blocked when SPV-T3a was added directly following the initiation of the MLC. When addition of SPV-T3a was postponed to one or more days following initiation, this effect gradually ceased to exist. Following four days, addition of SPV-T3a had no longer effect on proliferation. The irrelevant isotype-matched control antibody did not influence alloactivation at all time points. Translated to the *in vivo* situation these results demonstrate that unconjugated SPV-T3a is capable of delivering a direct and important immunosuppressive effect by preventing *ongoing allostimulation* of T lymphocytes. For the suppression or elimination of *already stimulated* T lymphocytes, SPV-T3a is dependent on another effector mechanism, termed activation induced cell death (AICD) (Appendix III, pg. 2), or needs to be conjugated to a toxin (Appendix III, pg 3).

### ACTIVATION INDUCED CELL DEATH BY UNCONJUGATED SPV-T3a

### Method:

Reduction of TCR-mediated cytotoxicity following IT-treatment was assayed *in vitro* using a cytotoxic T cell clone (CTL-clone) recognizing EBV-peptide EBNA3C presented in HLA-B44. CLT activity was assayed by lysis of a ⁵¹Cr-loaded EBV-transformed lymphoblastoide cell line (EBV-LCL) originating from the same individual. The CTL-clone was treated for 24 hours with SPV-T3a, washed and assessed either directly or following four days of additional incubation in culture medium. The extended four day incubation period was incorporated since during this time the CTL-clone restore its normal expression of the TCR/CD3 complex (which is blocked and or modulated directly following incubation with SPV-T3a.

### Results:

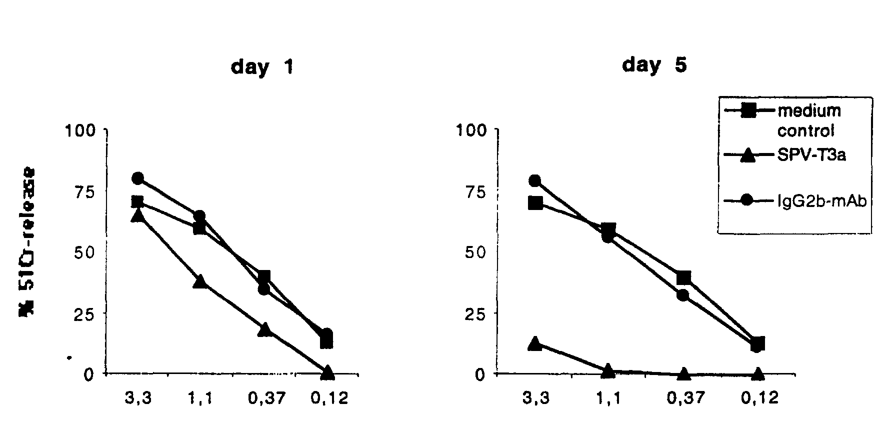

Directly following treatment (day 1), incubation with native mAb SPV-T3a (10⁻⁸M) resulted in a modest reduction of CTL-cytotoxicity. Flowcytometric analysis revealed that this effect was predominantly caused by the blocking and modulation of the TCR/CD3 complex due to binding of SPV-T3a. Following four days of extended incubation, the CTL-clone regained its normal TCR/CD3-expression, but CTL-cytotoxicity was further reduced to 18% of the untreated control (day 5). This time, flow cytometric analysis revealed that the majority of the CTL-cells had died due to apoptosis, according to the mechanism described as 'activation induced cell death' (AICD). Translated to the *in vivo* situation this means that unconjugated SPV-T3a is capable of delivering an important immunosuppressive effect by eliminating a significant fraction of activated T lymphocytes. The efficacy of SPV-T3a will be further enhanced when conjugated to a toxin like ricin A (Appendix III, pg. 3).

### COMMON IT-COCKTAIL SYNERGISM OF SPV-T3a-dgA & WT1-dgA

### Method:

PHA-stimulated PBL were treated with 10⁻⁸M IT for 24 h at 37°C, washed, and cultured for another four days at 37° in IT-free culture medium (to enable the IT to display their full toxicity). After this lag period, cells were incubated with 2 µg/ml propidium iodine (PI) (Molecular Probes, Junction City, OR) and 2 µg/ml calcein AM (Calc) (Molecular Probes) for 1 hour at RT. Samples were then analyzed on a Coulter Epics Elite (Coulter) flow cytometer equipped with a 40 mW Argon ion laser running at 15 mW. A longpass-filter of 610 nm was used for measurement of PI-fluorescence, a bandpass-filter of 525/30 nm for Calc-fluorescence. Overlap of the emission spectra of PI and Calc could be adjusted by electronic compensation using single-labeled samples. Samples were analyzed in triplicate using a minimum of 10,000 cells. Viable cells were identified as being PI-negative and Calc-positive. Prior to FCM analysis, a fixed amount of inert beads (DNA-check, Coulter) was added (10⁵ beads/ml) to enable the calculation of the number of surviving cells. The reduction of PBL was related to the viable fraction of the untreated control.

### Results:

| **Treatment** | **Factor of PBL reduction** |
|---|---|
| **- SPV-T3a-dgA** | **100** |
| **- WT1-dgA** | **87** |
| **- SPV-T3a-dgA and WT1-dgA** **(half a dose each)** | **1770** |

Due to the 'common IT-cocktail synergism' IT SPV-T3a-dgA and WT1-dgA appeared to be far more effective in combination (half a dose each) than either IT alone.

### REDUCTION OF NATURAL KILLER ACTIVITY BY WT1-dgRTA

### Method:

Blood mononuclear cells were isolated from peripheral blood by Ficoll centrifugation and incubated with 10⁻⁸M mAb or IT in a concentration of 1x10⁶/ml for 24 hrs. Subsequently, cells were washed and analyzed for NK-activity after 4 additional days of incubation without IT (this lag period is essential for IT to display their full efficacy). During the experiment, 50 units/ml recombinant IL₂ was added to the culture medium to increase NK-activity. For analysis of NK activity, cells were serial diluted and incubated with a fixed number of ⁵¹Cr-labeled K562 blasts (10⁴/100 µl) to yield an effector to target ratio of 10:1, 3.3:1, 1.1:1, and 0.37:1. After 3.5 hrs of incubation at 37°C, the cell mixtures were centrifuged and radioactivity was measured. NK-activity was expressed as percentage maximum ⁵¹Cr-release as determined with saponin treated ⁵¹Cr-labeled K562 blasts. Both were corrected for spontaneous ⁵¹Cr-release as determined with ⁵¹Cr-labeled K562 blasts incubated with culture medium only.

### Results:

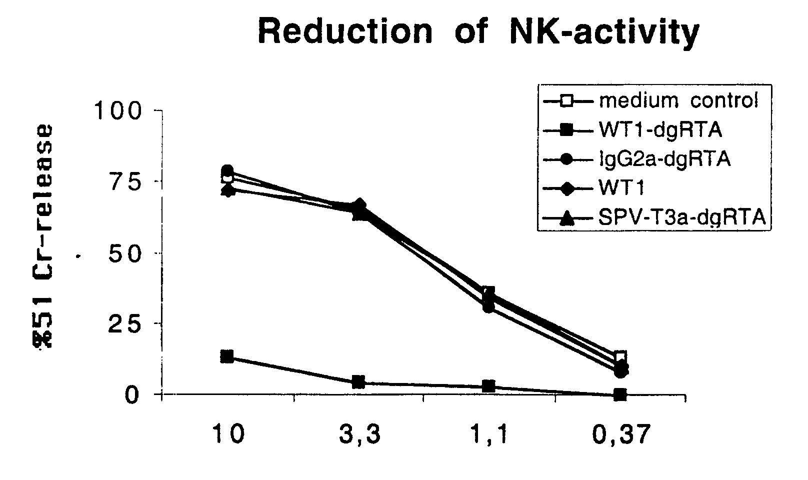

Incubation with saturating amounts of mAb SPV-T3a (10 µg/ml) had no effect on the NK-activity, nor had treatment with SPV-T3a-dgA (10⁻⁸ M). Four days following incubation with WT1-dgA, in contrast, the NK activity distinctively reduced to 8% of the untreated control. Unconjugated WT1, nor the isotype matched control IT, did influence the NK activity. Translated to the in vivo situation this means that incorporation of WT1-dgA in the IT-cocktail not only results in the 'common IT-cocktail synergism (Appendix III, pg. 3), but also broadens the spectrum reactivity. This is of vital importance since, though initiated by CTL, GVHD is thought to be aggravated by less specific cytokine-stimulated bystander cells like monocytes and NK/LAK cells.

### Animal toxicity studies:

A. LD50 determination with Balb/C mice:
   - IT-cocktail: 25-45 mg/kg
   - RFB4-dgA (based on literature): 14 mg/kg
B. Administration to Java-monkey's:
   - Rise of CK-levels following infusion
   - No further acute toxicities

### Determination of LD50:

| Survival Balb/C mice following i.v. administration IT-cocktail | | |
|---|---|---|
| Dose (µg/mouse) | Time of death | Survival after 2 weeks |
| 770 (n = 4) | day 0, day 9, day 9 | 25% |
| 385 (n = 3) | - | 100% |
| 193 (n = 4) | - | 100% |
| 96 (n = 4) | - | 100% |

percentage weight change following administration

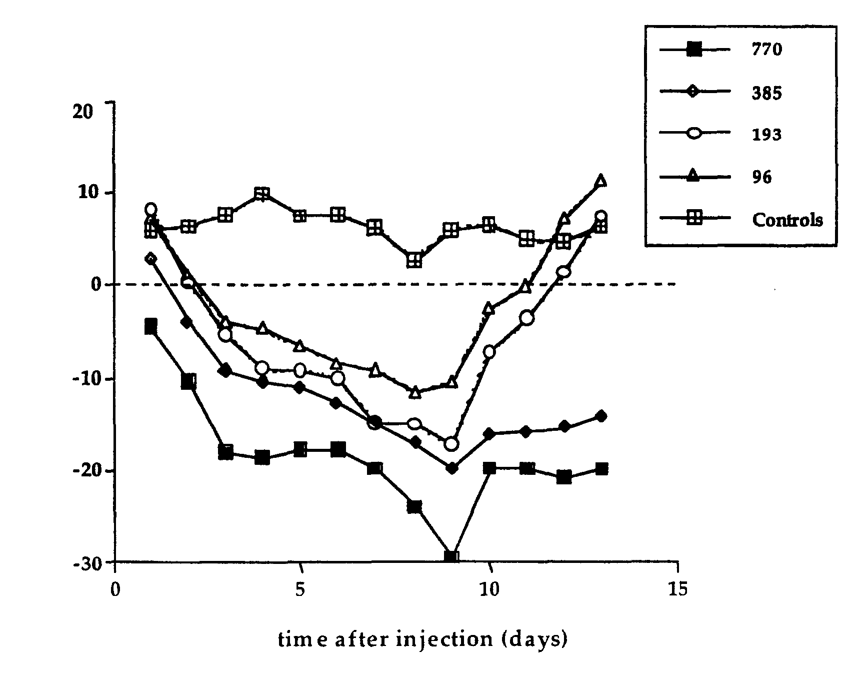

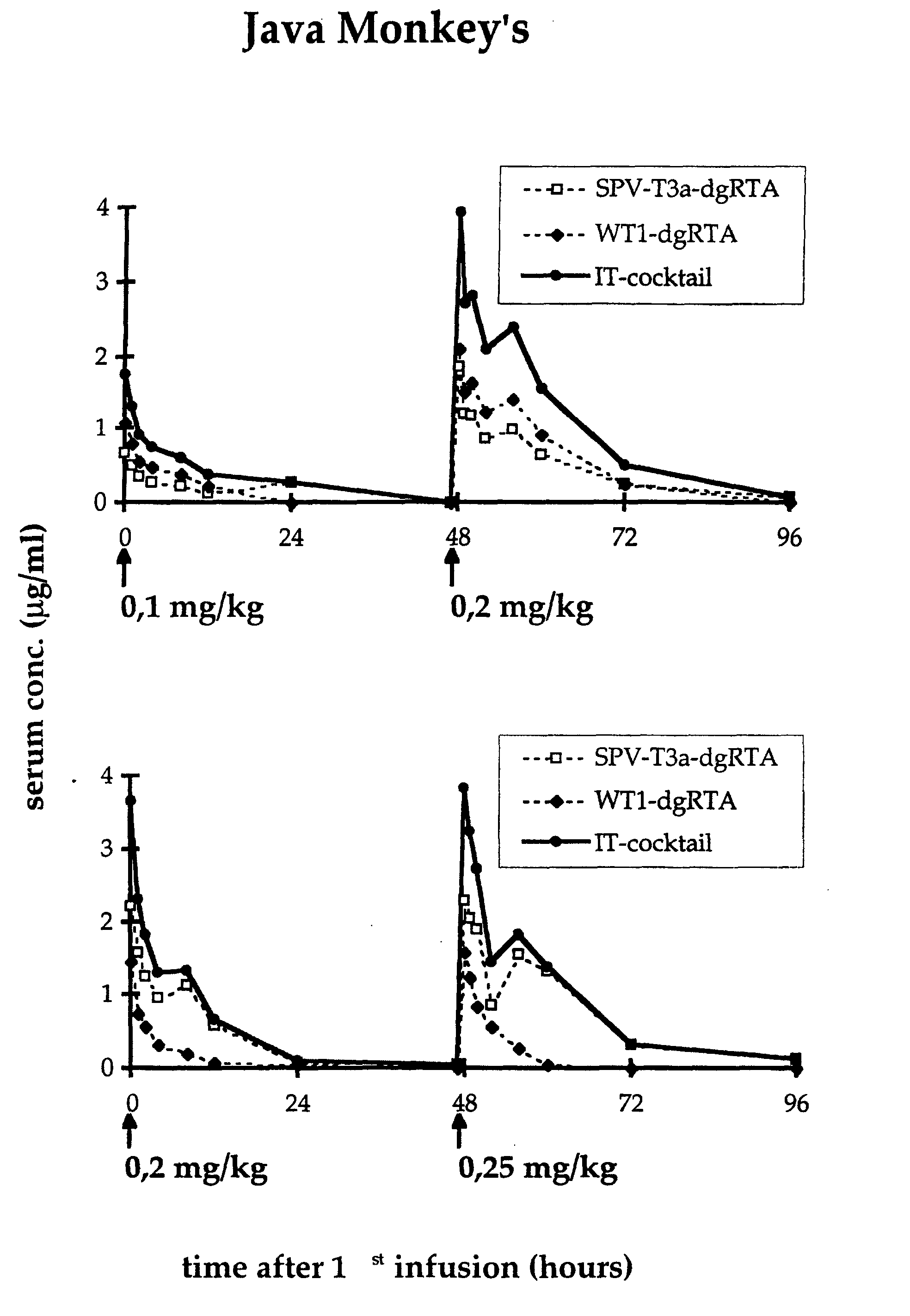

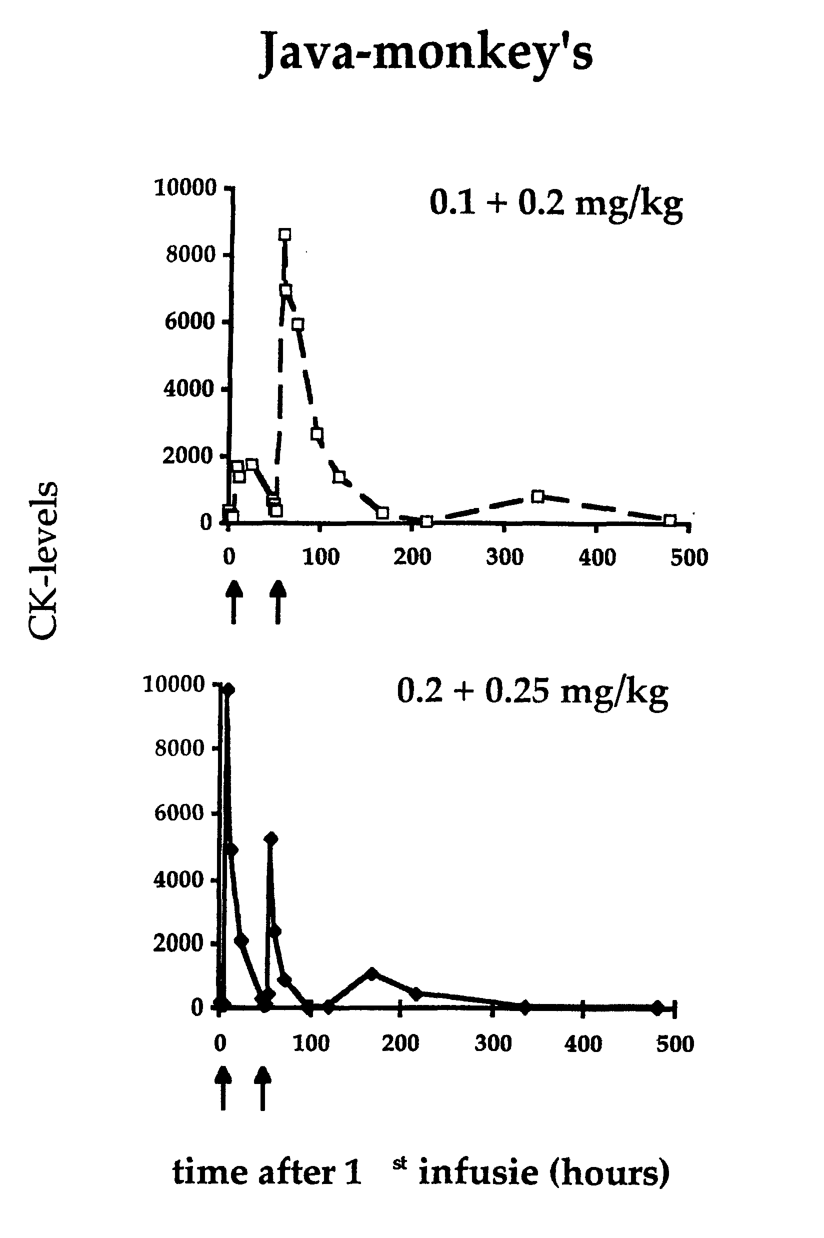

### Clinical pilot-study:

- Ongoing one center, non-randomized , open labeled, dose escalating study (aim of treating 5-7 patients)
- Four doses intravenously at 48-hours intervals:

| | Dosage of IT-cocktail (mg / m²) | | | | |
|---|---|---|---|---|---|
| #patients | d1 | d3 | d5 | d7 | Total |
| 1 | 2 | 2 | 4 | 4 | 12 |
| | | | | | |
| 2 | 4 | 4 | 4 | 4 | 164 |
| | | | | | |
| 2 | 8 | 8 | 8 | 8 | 32 |
| | | | | | |
| 2 | 10 | 10 | 10 | 10 | 40 |

- Evaluation: pharmacokinetics, toxicities, human-anti-mouse antibodies and human-anti-ricin antibodies (HAMA and HARA), biological and clinical responses

### First patient, characteristics:

- Male 60, Multiple Myeloma
- Sibling transplantation
- GVHD of skin, gut and liver (overall grade IV)
- Complication: multi-organ failure
- IT-cocktail: 2 doses 2 mg/m², 1 of 4 mg/m²
- Died seven day's following the first infusion

### First patient, toxicities:

- Mild capillary leakage, no weight gain
- No increase of CK-levels
- No further acute toxicity's
- No HAMA's/HARA's

### First patient, clinical respons:

- Skin: improvement starting at day 5
- Liver: stable (poor condition)
- Gastrointestinal tract: not interpretable (morfine)

### First patient, biological respons:

- Impressive reduction of circulating T/NK cells (CD2⁺5⁺ and CD2⁺, respectively) 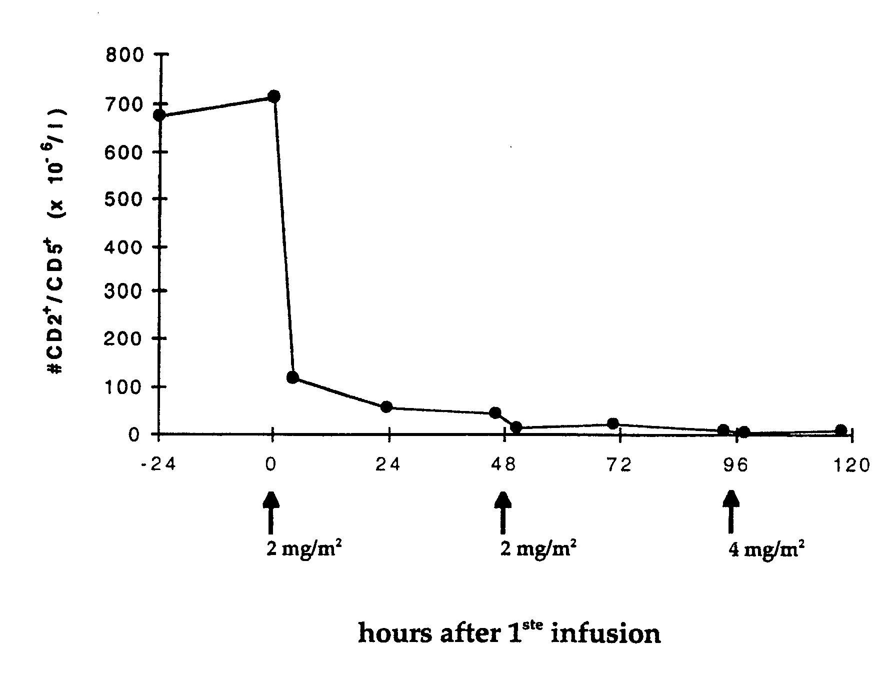
- During first 4 hour infusion: decrease to 17%
- Gradually declines further to 1% at day 7
- Dual mechanism: mAb-based (fast) & dgA-based (lasting)

### Second patient, characteristics:

- Male 34, CML
- Matched unrelated donor
- Grade 4 GVHD of the skin
- IT-cocktail: 2 doses 2 mg/m², 2 of 4 mg/m²

### Second patient, toxicities:

- No acute toxicity's could be observed
- No HAMA's/HARA's

### Second patient, responses:

- Dramatic (complete) response starting at day 5
- Lasting for ∼ 1.5 month
- Relapse of GVHD I-II
- Responding to low dose corticosteroids
- Died 8 months following treatment due to an inf ection

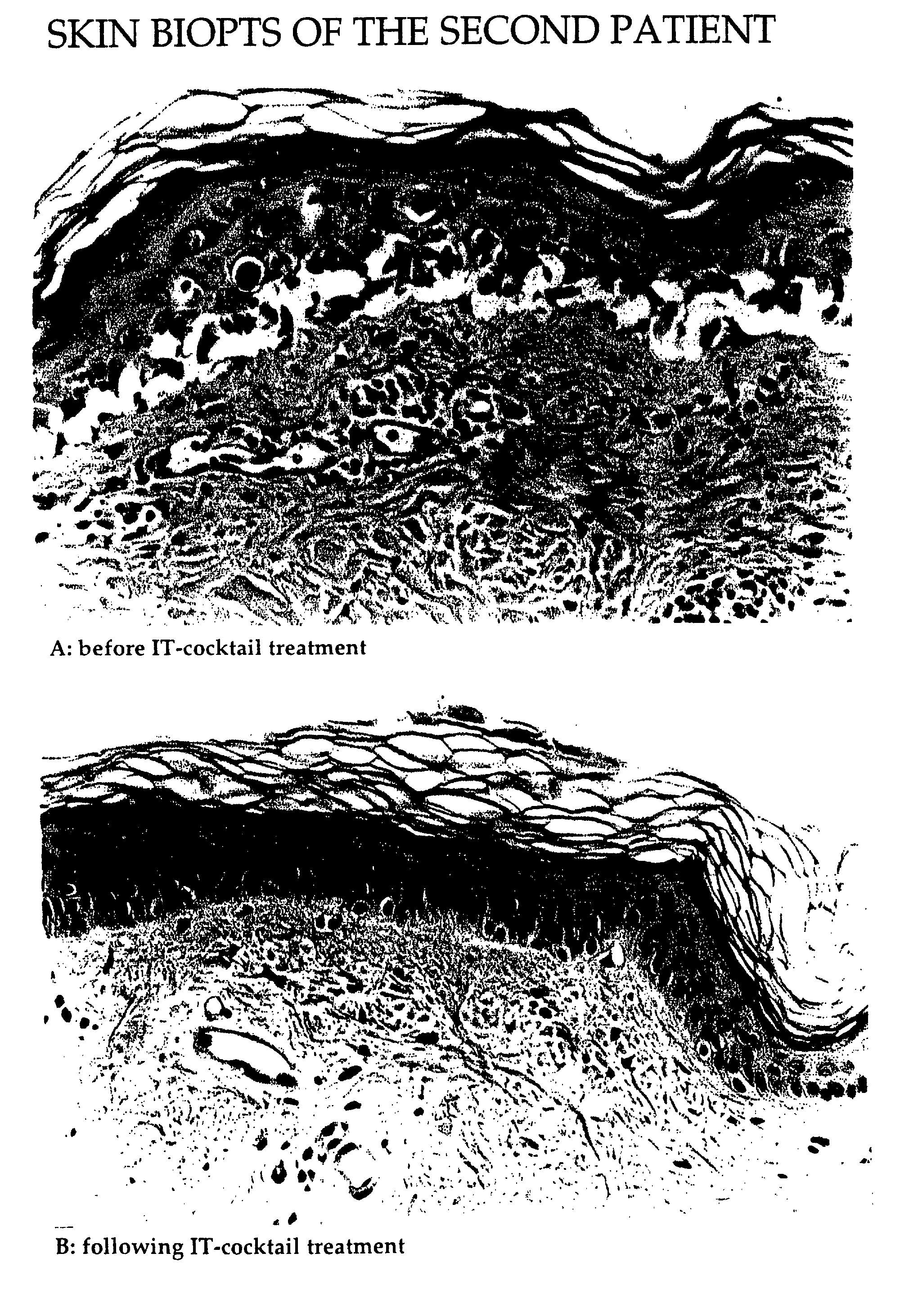

Microscopic analysis of the second patients skin-biopts taken just before (A), and two weeks following (B), treatment with IT-cocktail. The appearance of the skin before treatment is typical for severe GVHD (A). There is a massive infiltration of lymphocytes, which are localized primarily around the blood vessels and the joining region of the dermis (lower side) and epidermis (upper side). The basal membrane of the epidermis is destroyed, resulting in the epidermis coming of the dermis. Within two weeks following treatment, a dramatic clinical improvement of the skin could be observed. This was reflected by the microscopic analysis (B): there were no remaining signs of lymphocyte infiltration, the epidermis was lying close to the dermis, and the basal membrane had regained its well-organized natural configuration.

### Third patient, characteristics:

- Male 47, MDS
- HLA-identical donor
- GVHD grade 3/4 of the gut
- IT-cocktail: 4 doses of 4 mg/m²

### Third patient, toxicities:

- Rise bodytemp during infusions
- No further acute toxicities
- No HAMA's/HARA's

### Third patient responses:

- Reduction of lymphocytes 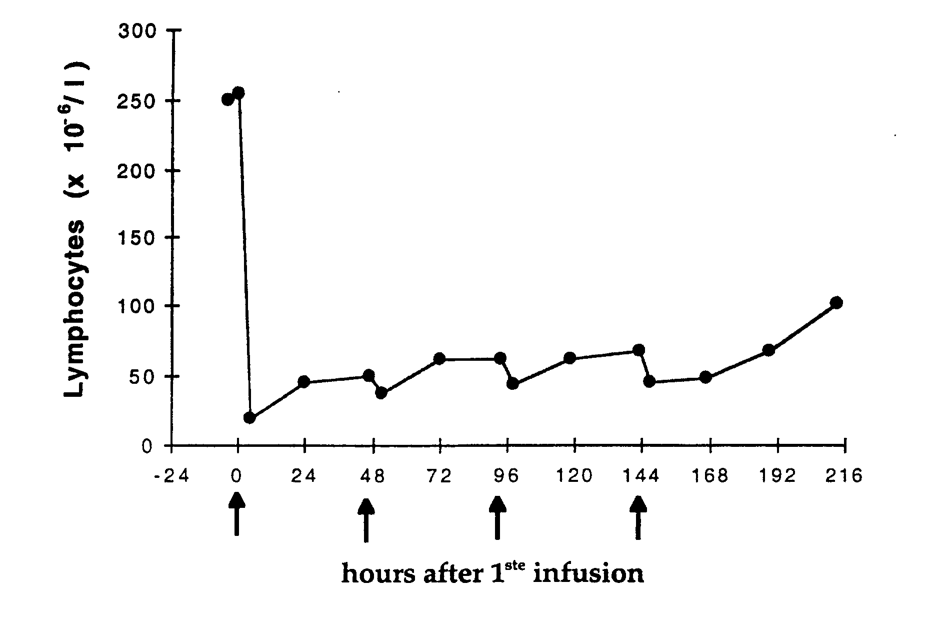
- Decrease of stool volume
- Endoscopy: strong improvement of gut-tissue

### Conclusions pilot-study:

- IT-cocktail is well tolerated, no acute severe toxicities
- extensive biological and clinical responses in the absence of acute severe toxicities
- IT-cocktail forms effective tool for in vivo suppression or elimination of misdirected, over-reactive or malignant T cells and / or NK-cells

## Claims

1. Use of a composition comprising a mixture of at least a first molecule specifically recognizing CD3 and at least a second molecule specifically recognizing CD7, whereby at least one of the specifically recognizing molecules is provided with a toxic moiety, in the preparation of a medicament for *in vivo* suppression or elimination of misdirected, over-reactive or malignant T cells and/or NK-cells, for the treatment of Graft vs. Host disease, Graft rejections, T-cell leukemia's, T-cell lymphomas or other CD3 and/or CD7 positive malignancies, autoimmune diseases or infectious immune diseases such as HIV-infection.

2. Use according to claim 1, whereby said first molecule and said second molecule are provided with a toxic moiety.

3. Use according to claim 1 or 2, wherein said first and/or said second molecule is an antibody, or a fragment or a derivative thereof.

4. Use according to any one of claims 1-3, wherein said mixture comprises at least one further molecule specifically recognizing CD5, CD2, CD4, CD8 or an IL-2 receptor.

5. Use according to claim 1, wherein said first molecule is a gamma2B IgG antibody or a derivative thereof, which recognizes CD3.

6. A kit of pharmaceutical compositions for treating Graft vs. Host disease and/or graft rejection comprising a corticosteroid and a mixture of at least a first molecule specifically recognizing CD3 and at least a second molecule specifically recognizing CD7, whereby at least one of the specifically recognizing molecules is provided with a toxic moiety.

## Patentansprüche

1. Verwendung einer Zusammensetzung, welche eine Mischung aus mindestens einem ersten Molekül, das spezifisch CD3 erkennt, und mindestens einem zweiten Molekül, das spezifisch CD7 erkennt, enthält, wobei mindestens eines der spezifisch erkennenden Moleküle mit einem toxischen Teil ausgestattet ist, zur Herstellung eines Medikaments zur Suppression oder Elimination von fehlgeleiteten, überreaktiven oder malignen T-Zellen und/oder NK-Zellen *in vivo*, für die Behandlung von Graft-vs-Host-Leiden, Gewebeabstossungen, T-Zell-Leukämien, T-Zell-Lymphomen oder anderen CD3- und/oder CD7-positiven, bösartigen Erkrankungen, Autoimmunerkrankungen oder infektiösen Immunkrankheiten wie etwa HIV-Infektion.

2. Verwendung gemäss Anspruch 1, worin das erste und das zweite Molekül mit einem toxischen Teil ausgestattet sind.

3. Verwendung gemäss Anspruch 1 oder 2, worin das erste und/oder zweite Molekül ein Antikörper oder ein Fragment oder Derivat davon ist.

4. Verwendung gemäss einem der Ansprüche 1 bis 3, worin die Mischung mindestens ein weiteres Molekül enthält, welches spezfisch CD5, CD2, CD4, CD8 oder einen IL-2-Rezeptor erkennt.

5. Verwendung gemäss Anspruch 1, worin das erste Molekül ein gamma2B IgG-Antikörper oder ein Derivat davon ist, welches CD3 erkennt.

6. Ein Kit pharmazeutischer Zusammensetzungen zur Behandlung von Graft-vs-Host-Leiden und/oder Gewebeabstossung, mit einem Corticosteroid und einer Mischung aus mindestens einem ersten Molekül, das spezifisch CD3 erkennt, und mindestens einem zweiten Molekül, das spezifisch CD7 erkennt, wobei mindestens eines der spezifisch erkennenden Moleküle mit einem toxischen Teil ausgestattet ist,

## Revendications

1. Emploi d'une composition comprenant un mélange d'au moins une première molécule reconnaissant spécifiquement CD3 et au moins une seconde molécule reconnaissant spécifiquement CD7, avec pour effet qu'au moins une des molécules reconnaissant spécifiquement est pourvue d'une fraction toxique, dans la préparation d'un médicament pour la suppression ou l'élimination *in vivo* de cellules mal dirigées, surréactives ou malignes T et/ou de cellules NK pour le traitement de greffe par rapport à une maladie de l'hôte, les réjections de greffe, les leucémies des cellules T, les lymphomies de cellules T ou autres maladies autoimmunes, malignes positives de CD3 et/ou de CD7 ou des maladies immunes infectieuses telle que infection par le SIDA.

2. Emploi selon la revendication 1, avec pour effet que ladite première molécule et ladite seconde molécule sont pourvues d'une fraction toxique.

3. Emploi selon la revendication 1 ou 2, dans lequel ladite première et/ou ladite seconde molécule est un anticorps, ou un fragment ou un dérivé de celui-ci.

4. Emploi selon l'une quelconque des revendications 1 à 3, dans lequel ledit mélange comprend au moins une autre molécule reconnaissant spécifiquement CD5, CD2, CD4, CD8 ou un récepteur IL-2.

5. Emploi selon la revendication 1, dans lequel ladite première molécule est un anticorps gamma 2B IgG ou un dérivé de celui-ci qui reconnaît CD3.

6. Kit de composition pharmaceutique pour traiter la greffe par rapport à une maladie de l'hôte et/ou la réjection de greffe comprenant un corticostéroide et un mélange d'au moins une première molécule reconnaissant spécifiquement CD3 et d'au moins une seconde molécule reconnaissant spécifiquement CD7, avec pour effet qu'au moins une des molécules reconnaissant spécifiquement est pourvue d'une fraction toxique.
